# EUROPÄISCHE PATENTANMELDUNG

(11) **EP 1 442 743 A1**
(43) Veröffentlichungstag der Anmeldung: **04.08.2004**
(21) Anmeldenummer: 04001224.7
(22) Anmeldetag: 21.01.2004
(51) Int. Cl.: A61K 31/385, A61K 31/137, A61K 31/401, A61P 25/28

(54) **Alpha-Liponsäure, Ambroxol und/oder Inhibitoren des Angiotensin Converting-Enzyms (ACE) umfassende Arzneimittel-Zubereitung und ihre Verwendung zur Behandlung von neurodegenerativen Erkrankungen**

(30) Priorität: 28.01.2003 DE 10303229
(71) Anmelder: KeyNeurotek AG, 39120 Magdeburg (DE); IMTM GmbH, 39120 Magdeburg (DE)
(72) Erfinder: Ansorge, Siegfried, Prof. Dr., 39291 Hohenwarte (DE); Röhnert, Peter, Dr., 39112 Magdeburg (DE); Striggow, Frank, Dr., 39174 Gerwisch (DE); Täger, Michael, Dr., 39326 Heinrichsberg (DE); Reymann, Klaus, Prof. Dr., 39167 Niederndodeleben (DE); Schröder, Ulrich, Dr., 97776 Eussenheim (DE)
(74) Vertreter: Koepe, Gerd L., Dipl.-Chem.

(57) **Zusammenfassung**

Die vorliegende Erfindung betrifft die Anwendung einer Medikamentenkombination zur Prävention und zur Therapie von neurodegenerativen Erkrankungen (z.B. ischämischer und hämorrhagischer Schlaganfall, fokale und globale Ischämie, amyotrophe Lateralsklerose (ALS), Alzheimer'sche Erkrankung, Parkinson'sche Erkrankung, Hunntington'sche Erkrankung, Multiple Sklerose). Bei der Wirkstoffkombination handelt es sich mindestens um zwei der folgenden Substanzen: α-Liponsäure; Ambroxol und ein oder mehrere Inhibitor(en) des Angiotensin-Converting Enzyms (ACE). Es konnte in den der Erfindung zugrunde liegenden Experimenten überraschenderweise gefunden werden, daß neuronale Zellen, die besonders empfindlich gegenüber degenerativen Insulten sind, weniger freie konstitutionelle Thiolgruppen aufweisen, als dies für andere Zellen des Zentralnervensystems (ZNS) der Fall ist. Es wurde demonstriert, daß dieser verminderte Gehalt an freien Thiolgruppen ursächlich mit der Schädigung dieser Zellen nach degenerativem Ereignis zusammenhängt. Durch die Verwendung der Kombination von α-Liponsäure, Ambroxol und/oder eines ACE-Inhibitor konnte der Schaden nach neurodegenerativem Insult um 40 bis 45 % vermindert werden.

## Beschreibung

Die Erfindung betrifft eine Arzneimittel-Zubereitung, die α-Liponsäure, Ambroxol und/oder einen ACE-Inhibitor (Inhibitor des Angiotensin-Converting-Enzyms) umfaßt. Die Erfindung betrifft weiter die Verwendung einer Arzneimittel-Zubereitung zur therapeutischen Behandlung von degenerativen Erkrankungen des Zentralnervensystems (ZNS), wie z.B. ischämischer und hämorrhagischer Schlaganfall, fokale und globale Ischämie, amyotrophe Lateralsklerose (ALS), Alzheimer'sche Erkrankung, Parkinson'sche Erkrankung, Hunntington'sche Erkrankung, Multiple Sklerose, Neurodegeneration im Alter und altersbedingter Demenz, Trauma und Autosomal Dominanter Neurohypophysealer Diabetes Insipidus (ADNDI). Eingeschlossen ist dabei auch die Verwendung der Zubereitung gemäß der Erfindung zur Prävention und Therapie zerebraler Ischämie infolge von kardialen und kardiovaskulären Insulten. Die genannten klinischen Indikationen führen zum Funktionsverlust von bestimmten Arealen des ZNS und resultieren in fast allen Fällen in einer irreversiblen geistigen und körperlichen Behinderung. Ein regulativer Eingriff in den zellulären Thiolstatus soll im Falle von neurodegenerativen Erkrankungen zum einem die Ausbildung des Schadens minimieren und zum anderen bei leichter geschädigten Zellen in der Penumbra dem Schaden vorbeugen.

Der zelluläre Thiol/Disulfidstatus ist eine der wichtigsten Grundvoraussetzungen biologischer Stoffwechselleistungen. Insbesondere wird durch die Oxidation und Reduktion von Thiol/Disulfidgruppen die Funktionalität von Proteinen in großem Maße beeinflußt.

Der durch verschiedene Enzymklassen regulierte Metabolismus der Disulfidspaltung und Thiolgruppenbildung ist durch die Vielfalt seiner biologischen Funktionen u. a. bei zellulären Wachstums- und Differenzierungsprozessen einschließlich des programmierten Zelltodes sowie Zellschutzund Entgiftungsmechanismen in seiner Intaktheit unabdingbar für die Vitalität einer Zelle. Störungen in diesem System und Veränderungen der Konzentration der Thiole führen zu schwerwiegenden zellulären Funktionsstörungen, die nur im Einzelfall lokal begrenzt bleiben, in der Regel jedoch große Teile der betroffenen und angrenzenden Gewebe bzw. den gesamten Organismus beeinträchtigen.

Die Rolle eines gestörten Thiol/Disulfidstatus in der Zelle sowohl bei der Ausbildung als auch bei der Progression neurodegenerativer Erkrankungen wurde bislang nur unzureichend untersucht. Es besteht daher der dringende Bedarf, die Verknüpfungen von Thiolstatus und der Pathophysiologie neurodegenerativer Erkrankungen aufzuklären, v.a. vor dem Hintergrund der Findung potenter Wirkstoffe zur Behandlung von Erkrankungen und Verletzungen des ZNS, bei denen bislang eine adäquate medikamentöse Intervention nicht verfügbar ist.

Bei der Degeneration von Neuronen nach ischämischen Ereignissen spielt neben der Schädigung des Endothels (Veränderung der Barrierefunktion der Blut-Hirn-Schranke) auch die Inhibition endogener Schutzmechanismen eine Rolle, da viele Enzyme und Regenerationssysteme infolge der Ischämie inhibiert sind [1, 2]. Dies macht sich v.a. in der Reperfusionsphase bemerkbar.

Nicht nur beim Schlaganfall, sondern auch bei anderen neurodegenerativen Erkrankungen (sowohl mit akuten als auch chronischen Verläufen), wie z.B. Parkinson'sche Erkrankung, Alzheimer'sche Krankheit [3], Alterungsprozesse [4, 5], hämorrhagischer Schlaganfall [6], Exzitotoxizität [7], ALS und Trauma [8], wird eine neuronale Schädigung durch fehlende bzw. inhibierte Schutz- und Regenerationssysteme diskutiert. Zu welchem Anteil, ist allerdings bislang ungeklärt. Sind nun Teilkomponenten dieser endogen Schutzsysteme defekt oder, wie am Beispiel der Pyramidenzellen hier gezeigt, nur in geringem Maße vorhanden, kann dies dramatische Folge haben.

Fehlende zelluläre Schutzmechanismen führen u.a. zur Lipidperoxidation, in deren Folge die Integrität zelluläre Membranen verloren geht. Betroffen sind dadurch u.a. membranständige Proteine. Beispielsweise sind bei Ischämie Glutamattransporterproteine funktionell beeinträchtigt [8], deren Funktion darin besteht, freigesetztes Glutamat wieder in die Zelle zu transportieren, und damit toxische extrazelluläre Konzentrationen dieses Neurotransmitters zu verhindern.

α-Liponsäure wird bislang mit relativem Erfolg zur Behandlung von Mißempfindungen im Rahmen der peripheren diabetischen Polyneuropathie als Adjuvans eingesetzt (Diabetologica 1995; 38:1425-1433, Diabetes Res Clin Pract 1995;29:19-26, Diab Care 1999;22:1296-1301, Drug Metab Rev 1997; 29:1025-1054, DE 43 43 592 C2). In den Patentschriften DE 44 47 599 C2 und EP 0 530 446 B1 ist darüber hinaus die Verwendung von α-Liponsäure bei weiteren neuronalen Störungen einschließlich Tinnitus und Hörsturz geschützt.

Der zytoprotektive Wirkmechanismus bei diabetischen Begleiterscheinungen beruht neben der Beeinflussung der zuckerabhängigen Proteinmodifkation (Proteinglykosylierung) sowie einer Verringerung der toxischen Ketonkörper-Genese auch auf der antioxidativen Funktion der α-Liponsäure und deren Metabolite [9].

In den Patentschriften EP 0 812 590 A2 sowie EP 0 427 247 B1 wird die Verwendung von α-Liponsäure als peripheres Zytoprotektivum, Antischmerzmittel sowie als Medikament bei Entzündungserkrankungen offenbart.

Ambroxol, (trans-4-(2-Amino-3,5-dibromobenzyl-amino)-cyclohexan-hydrochlorid) wird in verschiedenen Darreichungsformen bei Lungen- und Bronchialerkrankungen als schleimlösendes Medikament eingesetzt (WO 96/33704, GB 2,239,242, WO 01/05378). Darüber hinaus ist die Verwendung bei Hyperurikämie aus der DE 35 30 761 bekannt. Die Wirkung des Ambroxols als Mukolytikum beruht sowohl auf einer Stimulation der Surfactant-Produktion der Bronchialzellen als auch insbesondere auf der Fähigkeit, freie Radikale zu eliminieren [10]. Die hierauf basierende antioxidative Aktivität der Substanz wurde hauptsächlich an pulmonalen Zellen [11] aber auch im Rahmen von entzündlichen Mechanismen nachgewiesen [12]. Weiterhin ist bekannt, daß durch den Zusatz von Ambroxol in hohen Dosen regulatorische Enzyme des Glutathionstoffwechsels direkt beeinflusst werden [13].

Inhibitoren des angiotensin converting enzymes (ACE) werden mit großem Erfolg bei der Behandlung einer breiten Palette kardiovaskulärer Erkrankungen eingesetzt. Diese Substänzklasse vermittelt ihre blutdrucksenkende Wirkung über die Hemmung der Umwandlung von Angiotensin-I in Angiotensin-II bzw. über die Beeinflussung des Kinin-Stoffwechsels. Für die Beeinflussung von intrazellulären Redoxprozessen sind die Wirkungen Thiol-Gruppen tragender ACE-Inhibitoren, z.B. Captopril (1-[(2S)-3-Mercapto-2-methylpropion-yl]-L-prolin), von denen Thiol-freier ACE-Inhibitoren, z.B. Enalapril (1-{N-[(S)-1-Ethoxycarbonyl-3-phenylprop-yl]-L-alanyl}-L-prolin), zu unterscheiden. Erstere reagieren direkt als Radikalfänger und damit antioxidativ, während SH-freie ACE-Inhibitoren dazu primär nicht in der Lage sind. Beiden Gruppen gemeinsam ist jedoch die Beeinflussung des Glutathionredoxzyklus über die Regulation der Glutathionreduktase, der Glutathionperoxidase sowie der Superoxiddismutase (Am J Physiol Regulatory Integrative Comp. Physiol.2000;278:572-577).

In der Druckschrift DE 44 20 102 A1 wurde bereits die Anwendung verschiedener Arzneimittel in Kombination mit α-Liponsäure zur Verbesserung bisheriger Arzneimitteltherapien bei kardiovaskulären und diabetisch bedingten Erkrankungen geschützt. Die Ansprüche dieser Patentschrift beschränken sich auf diese Anwendungsgebiete, mit der Ausnahme der Kombination von α-Liponsäure mit Calciumantagonisten, die nicht Bestandteil der hier beschriebenen neuartig gefundenen Substanzkombinationen sind. In der Druckschrift DE 44 20 102 A1 wird ausschließlich die Anwendung der Kombination α-Liponsäure und Calciumantagonisten für neurodegenerative Erkrankungen beansprucht. Gegenstand und Hauptanspruch von DE 44 20 102 A1 ist die Unterscheidung von verschiedenen Stereoisomeren der α-Liponsäure als differenziert einsetzbare Wirkstoffe sowie spezielle Arzneimittel-Formulierungen.

Im Gegensatz dazu werden in der vorliegenden Erfindung neuartige Substanzkombinationen und daraus formulierte Arzneimittel zur Verminderung neuronaler Schädigungsprozesse als Ursache und Folge von neurodegenerativen Erkrankungen beansprucht. Die Entwicklung von neuartigen Neuroprotektiva erfolgt auf der Basis von Substanzen, die den Thiol/Disulfidstatus von Zellen im ZNS positiv beeinflussen. Da zur Zeit keine valide medikamentöse Prävention und Therapie von neurodegenerativen Erkrankungen zur Verfügung steht (therapeutisch wie symptomatisch), eröffnet die hier beschriebene Erfindung möglicherweise ein großes pharmazeutisches Potential.

Grundlage der vorliegenden Erfindung ist nämlich der neuartige Befund, daß Neuronen mit einem geringeren Gehalt an freien (reduzierten) Thiolgruppen ausgestattet sind, was ursächlich deren Vulnerabilität gegenüber degenerativen Insulten negativ beeinflußt. Überraschenderweise konnte gezeigt werden, daß durch die Kombination von 2 oder mehreren Substanzen aus der Gruppe α-Liponsäure, deren Salze und Isomere, Ambroxol und dessen Salze und Prodrugs und mindestens einem Inhibitor des ACE eine deutliche Verminderung zerebraler Schäden nach degenerativen Ereignissen/Insulten erreicht werden kann. Damit ist möglicherweise ein erfolgversprechender Ansatz zur Prävention und Therapie von bisher nicht oder nur unzureichend behandelbaren neurodegenerativen Erkrankungen gefunden worden.

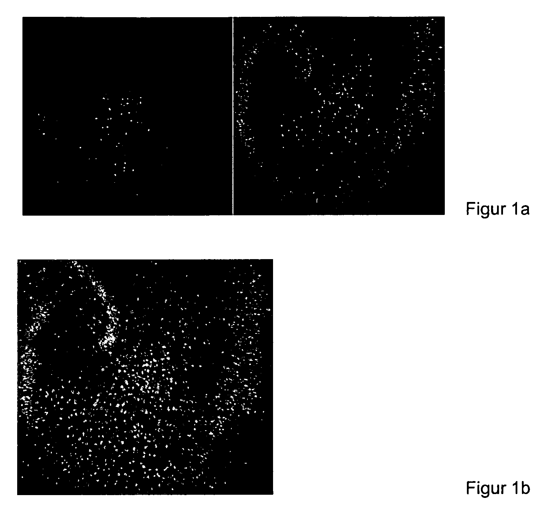

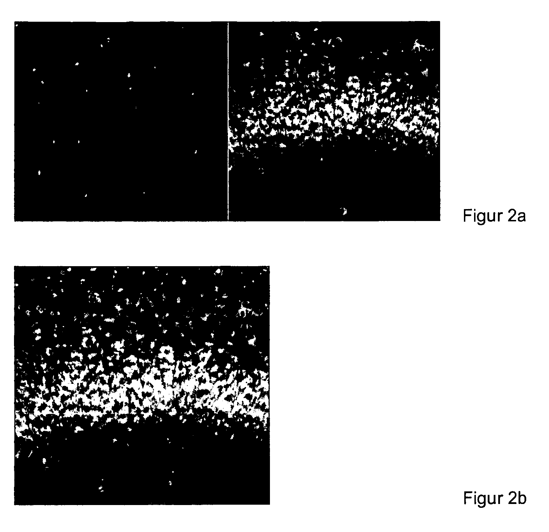

Im Rahmen der zu der vorliegenden Erfindung führenden Untersuchungen konnte der überraschende Zusammenhang gefunden werden, daß der intrazelluläre Thiol/Disulfidstoffwechsel eine erhebliche Rolle bei der Pathogenese neurodegenerativer Erkrankungen spielt. Es konnte in den vorliegenden Versuchen demonstriert werden, daß die äußerst vulnerablen Nervenzellen (Neuronen) des ZNS mit einem geringeren Gehalt an freien Thiolen ausgestattet sind als weniger vulnerable nichtneuronale Zellen (z.B. Astrozyten und Gliazellen). Sehr deutlich ist dies in den histochemischen Fluoreszenzaufnahmen der Abbildung 1a, b zu sehen, bzw. auf neuronaler Ebene in der Abbildung 2a, b. Weiterhin konnte aufbauend auf diesem Befund durch die gezielte Beeinflussung des zellulären Thiol-/Disulfidstatus mit zwei oder mehr Substanzen aus der Gruppe α-Liponsäure, deren Salze und Isomere, Modulatoren des Glutathionstoffwechsels (Ambroxol und dessen Salze und Prodrugs und ein ACE-Inhibitor) die bedeutende Rolle thiolreaktiver Verbindungen als Schutzmechanismus bei Neurodegeneration herausgestellt werden.

Überraschenderweise gelang es bei den beschriebenen Versuchen durch die Kombination von zwei oder mehr Substanzen aus der Gruppe α-Liponsäure, deren Salze und Isomere, Ambroxol und dessen Salze und Prodrugs und mindestens ein ACE-Inhibitor das Überleben von Neuronen nach dem neurodegenerativen Insult signifikant zu verbessern. Im Gegensatz dazu führte die Applikation der Einzelsubstanzen zu keinem Effekt. Gemessen wurde in den vorliegenden Experimenten der Anteil an degenerierenden Neuronen, der einen klinisch außerordentlich relevanten Parameter darstellt.

Die Erfindung betrifft somit pharmazeutische Zubereitungen, die zwei oder mehr Substanzen aus der Gruppe α-Liponsäure und deren Salze und Isomere, Ambroxol und dessen Salze und Prodrugs und mindestens ein Inhibitor des Angiotensin-Converting-Enzyms (ACE) umfassen, gegebenenfalls zusammen mit üblichen pharmazeutisch annehmbaren Träger-, Zusatz- und Hilfsstoffen.

Die Erfindung betrifft auch die Verwendung einer derartigen, zwei oder mehr Substanzen aus der Gruppe α-Liponsäure und deren Salze und Isomere, Ambroxol und dessen Salze und Prodrugs und mindestens ein Inhibitor des Angiotensin-Converting-Enzyms (ACE), gegebenenfalls zusammen mit üblichen pharmazeutisch annehmbaren Träger- und Hilfsstoffen, umfassenden pharmazeutischen Zubereitung zur Prävention und Therapie von neurodegenerativen Erkrankungen.

Die Korrektur eines gestörten Thiolstoffwechsels durch α-Liponsäure, Ambroxol und/oder einem ACE-Inhibitor erlangt somit grundlegende Bedeutung für die Prävention und Therapie einer Vielzahl von Erkrankungen unterschiedlicher Genese, insbesondere jedoch unter dem Gesichtspunkt präventiver als auch therapeutischer Behandlungsmethoden von neurodegenerativen Erkrankungen. Ohne an dieser Stelle auf eine Theorie fixiert werden zu wollen, wird angenommen, daß die Wirkung der hier beschriebenen Verbindungen möglicherweise auf deren Thiol-stabilisierende Wirkung zurückzuführen ist, wobei durch die neuartige und gezielte Kombination der Wirkstoffe überraschenderweise synergistische neuroprotektive Effekte induziert werden konnten. Im Gegensatz dazu war die Applikation von Einzelsubstanzen grundsätzlich wirkungslos.

Die gewählten experimentellen Bedingungen entsprachen sehr weitgehend einer klinischen Situation [14, 15]. Bei allen getesteten Verbindungen handelt es sich um bereits zugelassene Arzneimittel. Der Nachweis der Unbedenklichkeit der Einzelsubstanzen ist demnach bereits erbracht. Sowohl Einzelsubstanzen als auch Substanzkombinationen zeigten keinerlei neurotoxische Eigenschaften.

Erfindungsgemäß werden die pharmazeutischen Zubereitungen mit den Merkmalen des Patentanspruchs 1 beansprucht. Mit anderen Worten: Die Erfindung umfaßt die Kombination sowohl zweier beliebiger als auch aller drei Wirkstoffe. Vorteilhafte Ausführungsformen ergeben sich aus den Unteransprüchen 2 bis 9. Patentanspruch 10 beansprucht die Verwendung der erfindungsgemäßen Zubereitungen in einem Indikationsgebiet. Vorteilhafte Ausführungsformen dieser Verwendungen ergeben sich aus den abhängigen Ansprüchen 11 und 12.

Eine Komponente der erfindungsgemäßen pharmazeutischen Zubereitungen kann α-Liponsäure sein. Unter dem Begriff "α-Liponsäure" wird erfindungsgemäß sowohl die reine Verbindung α-Liponsäure, terminologisch geführt unter der Bezeichnung (R)-5-(1,2-Dithiolan-3-yl-)pentansäure, als auch ihre Salze verstanden, wobei im vorliegenden Fall natürlich nur pharmazeutisch annehmbare Salze in Frage kommen. Unter "α-Liponsäure" wird erfindungsgemäß weiter das Stereoisomer verstanden, wie auch Mischungen der beiden Isomere, die das racemische Gemisch einschließen. Weiter wird erfindungsgemäß unter "α-Liponsäure" auch jeder Metabolit von α-Liponsäure verstanden, einschließlich oxidierter oder reduzierter Formen der genannten Säure. Es können auch Kombinationen der genannten α-Liponsäure-Substanzen verwendet werden.

Einer der verwendeten Effektoren des Glutathionmetabolismus und eine der in der erfindungsgemäßen pharmazeutischen Zubereitung verwendbaren Komponenten stellt Ambroxol der allgemeinen Formel I dar:

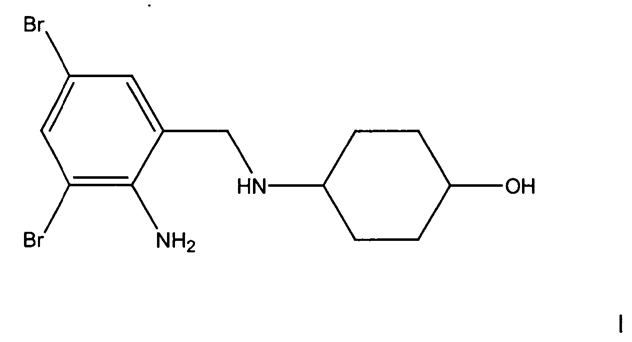

Ambroxol wird als solches oder - bevorzugt - in Form eines Salzes und/oder Prodrugs verwendet. Als Salze kommen nur pharmazeutisch anehmbare Salze in Frage. Unter "Prodrugs von Ambroxol" werden im Rahmen der vorliegenden Erfindung alle Substanzen verstanden, die sich als Vorstufe der Verbindung applizieren und/oder verabreichen lassen und im Körper - beispielsweise unter Enzymeinfluß - in den aktiven Wirkstoff umgewandelt (aktiviert) werden.

Als zweiter Effektor des Glutathionmetabolismus und weitere denkbare Komponente der erfindungsgemäßen pharmazeutischen Zubereitung wird ein Inhibitor (oder werden mehrere Inhibitoren) des Angiotensin-Converting-Enzyms (ACE) verwendet. In Frage kommen alle an sich bekannten Inhibitoren des Angiotensin-Converting-Enzyms. Beispiele sind Enalapril, Captopril, Lisopril, Ramipril und Spirapril, ohne die Erfindung auf diese zu beschränken. Mittlerweile sind mannigfaltige Molekülvarianten verfügbar, die sich bei gleichem pharmakodynamischen Profil vorwiegend in ihren pharmakokinetischen Eigenschaften unterscheiden.

Bei den erhobenen Befunden ist das Vorhandensein von freien Thiolgruppen im Molekül keine Voraussetzung für die Wirksamkeit, da bei Verwendung eines thiolgruppenfreien ACE-Inhibitors vergleichbare experimentelle Daten erbracht wurden. Es können erfindungsgemäß vorzugsweise die folgende Verbindungen eingesetzt werden:
A) 1-[(2S)-3-Mercapto-2-methylpropionyl]-L-prolin (Captopril) der Formel II 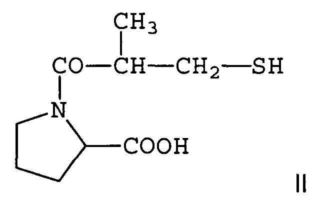
B) 1-{N²-[(S)-1-Carboxy-3-phenylpropyl]-L-lysyl}-L-prolin (Lisinopril) der Formel III 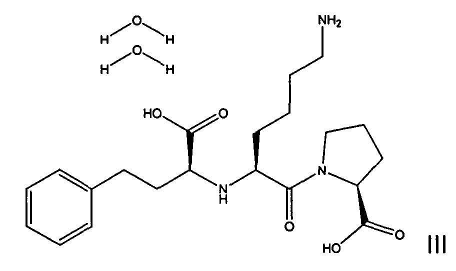
C) 1-{N-[(S)-1-Ethoxycarbonyl-3-phenylpropyl]-L-alanyl}-L-prolin (Enalapril) der Formel IV 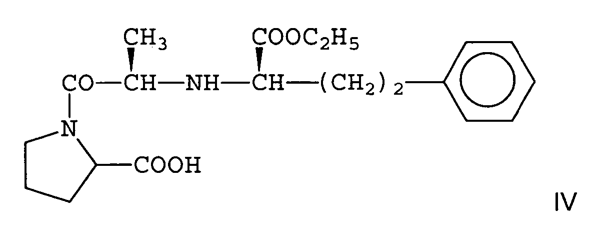
D) (2S,3aS,6aS)-1-{(S)-N-[(S)-1-Ethoxycarbonyl-3- phenylpropyl]-alanyl}-octahydrocyclopenta[b]- pyrrol-2-carbonsäure (Ramipril) der Formel V 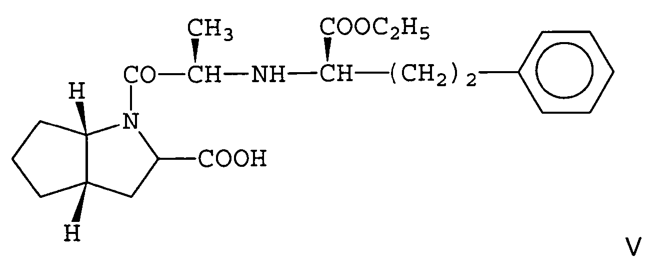
   Eingeschlossen sind z.B. auch Verbindungen bei denen Prolin subtituiert, modifiziert bzw. ersetzt wurde:
E) 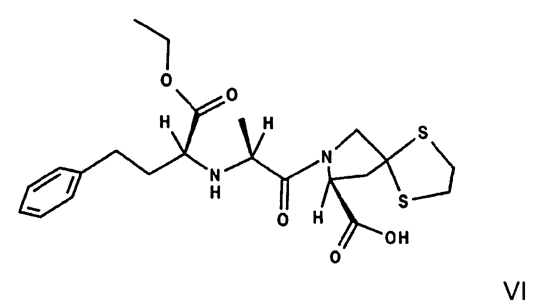 Spirapril (8S)-7-{(S)-N-[(S)-1-Ethoxycarbonyl-3-phenylpropyl]alanyl}-1,4-dithia-7-azaspiro[4,4]nonan-8-carbonsäure; 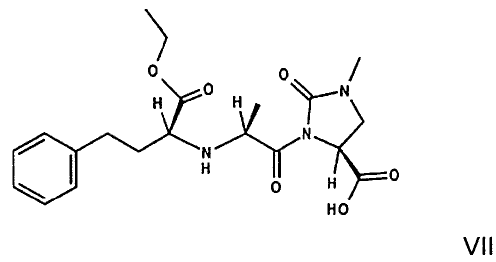 Imidapril (4S)-3-{N-[(S)-1-Ethoxycarbonyl-3-phenylpropyl]-L-alanyl}-1-methyl-2-oxo-4-imidazolidincarbonsäure; 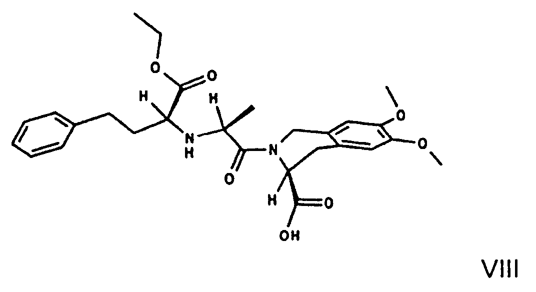 Moexipril (3S)-2-{N-[(S)-1-Ethoxycarbonyl-3-phenylpropyl]-L-alanyl}-1,2,3,4-tetrahydro-6,7-dimethoxy-3-isochinolincarbonsäure.

Im Fall dieser Verbindungen ist die bevorzugte Dosis für die humanmedizinische Applikation abhängig von der verwendeten Verbindung.

In den pharmazeutischen Zubereitungen gemäß der Erfindung können die Substanzen der genannten Gruppen in jeder beliebigen Kombination enthalten sein. Bevorzugte Beispiele gemäß der Erfindung sind pharmazeutische Kombinationspräparate, die α-Liponsäure, deren Salze, Stereoisomere oder Metabolite und Ambroxol, dessen Salze oder dessen Prodrug(s) in Kombination enthalten, oder die α-Liponsäure, deren Salze, Stereoisomere oder Metabolite und einen oder mehrere Inhibitoren des Angiotensin-Converting-Enzyms im Kombination enthalten, oder die einen oder mehrere Inhibitoren des Angiotensin-Converting-Enzyms und Ambroxol, dessen Salze oder dessen Prodrug(s) in Kombination enthalten, oder die α-Liponsäure, deren Salze, Stereoisomere oder Metabolite und Ambroxol, dessen Salze oder dessen Prodrug(s) und einen oder mehrere Inhibitoren des Angiotensin-Converting-Enzyms in Kombination enthalten.

Die Mengen, in denen die vorgenannten Komponenten in den erfindungsgemäßen Zubereitungen enthalten sind, sind in weiten Grenzen wählbar und richten sich in erster Linie nach den Gegebenheiten, die der Fachmann bei einem Patienten vorfindet, an den die Zubereitung verabreicht werden soll. Art und Außmaß der Krankheit, Status des Patienten, Gewicht und Stoffwechsel-Parameter des Patienten sowie der gewählte Verabreichungsweg stellen wichtige Variablen der zu verabreichenden Mengen dar. Die effektive Dosis von α-Liponsäure oder deren Salzen oder Isomeren ist - wie die effektive Dosis der anderen, in der erfindungsgemäßen pharmazeutischen Zubereitung enthaltenen Komponente(n) - fallbezogen zu ermitteln und richtet sich maßgeblich nach den üblichen Dosen für bekannte Anwendungsgebiete, wie auch nach patientenbezogenen Parametern, beispielsweise den vorgenannten Parametern.

In bevorzugten Ausführungsformen der Erfindung umfassen die Zubereitungen zur Verwendung bei der humanmedizinischen Applikation beim Patienten α-Liponsäure in Mengen zwischen 30 und 1.200 mg/d, besonders bevorzugt in Mengen zwischen 200 und 600 mg/d. Hierbei handelt es sich um Tagesdosen, die als Einmaldosis oder in beliebig vielen, ganz besonders bevorzugt in bis zu drei Einzeldosen pro Tag verabreicht werden können. Die Dosis von Ambroxol für die humanmedizinische Applikation liegt bevorzugt zwischen 7,5 und 90 mg/d, besonders bevorzugt zwischen 60 und 75 mg/d, wobei es sich ebenfalls um Tagesdosen handelt, die als Einmaldosis bzw. in beliebig vielen, ganz besonders bevorzugt in bis zu drei Einzeldosen pro Tag verabreicht werden können. Für die ACE-Inhibitoren liegt die Dosis für die humanmedizinische Applikation bevorzugt zwischen 1 und 50 mg/d, besonders bevorzugt zwischen 5 und 20 mg/d, wobei es sich ebenfalls um Tagesdosen handelt, die als Einmaldosis bzw. in beliebig vielen, ganz besonders bevorzugt in bis zu drei Einzeldosen pro Tag verabreicht werden können.

Zusätzlich können die pharmazeutischen Zubereitungen je nach Anwendungsbereich, Verabreichungsform oder aufgrund sonstiger Erfordernisse gängige Trägerstoffe, Zusatzstoffe und/oder Hilfsstoffe enthalten. Hierzu zählen z.B. wäßrige Lösungsmittel, Stabilisatoren, Suspensions-, Dispersions- und Benetzungsmittel, Sprengmittel (sofern eine orale Verabreichung mit Tabletten erfolgt) sowie Nahrungsergänzungsmittel.

Die erfindungsgemäßen pharmazeutischen Zubereitungen können nach an sich bekannten Verfahren in beliebiger Formulierung hergestellt werden. Beispielsweise gehören zu bevorzugten Formulierungen Lösungen, die organische und/oder anorganische Lösungsmittel enthalten können, Granulate, Aerosole, Pulver, Suspensionen, Emulsionen, (Kau-)Tabletten und/oder Filmtabletten, Retardtabletten, Kapseln sowie transdermale Applikationssysteme. Die Komponenten der erfindungsgemäßen pharmazeutischen Zubereitungen können dabei sowohl in einer einzigen Formulierung als auch in getrennten Formulierungen vorliegen.

Die erfindungsgemäß zur Anwendung kommenden Kombinationspräparate können in den üblichen pharmakologischen Darreichungsformen sowohl prophylaktisch als auch therapeutisch verabreicht werden. Denkbare Darreichungsformen sind: Tabletten (magensaftresistente Tablette, Filmtablette), Pulver, Granulate, Kapseln, Lösungen, Emulsionen, Suspensionen, Aerosole, transdermale Applikationssysteme, Darreichungsformen mit retardierter Abgabe einzelner oder aller Wirkstoffe oder solche in nicht retardierter Form. Die Komponenten können in räumlich getrennten Zubereitungen verabreicht werden. Es ist darüber hinaus auch möglich, diese getrennten Zubereitungen in einer Formulierung anzuwenden, mit der entsprechend fallbezogenen Konzentrationsmengen der Einzelkomponenten als Tabletten (magensaftresistente Tabletten Filmtabletten.), Pulver, Granulate, Kapseln, Lösungen, Emulsionen, Suspensionen, Aerosole, transdermale Applikationssysteme, oder als Darreichungsformen für die Wirkstoffabgabe in retardierter bzw. nicht retardierter Form verabreicht werden.

Die pharmazeutischen Zubereitungen gemäß der Erfindung können auf jedem beliebigem Verabreichungsweg verabreicht werden. Nicht beschränkende Beispiele hierfür sind die folgenden: orale, bukkale, pulmonale, nasale, transdermale, intravenöse, subcutane, intracutane, intramuskuläre, rektale, vaginale und intrathekale Verabreichung, gegebenenfalls zusammen mit an sich bekannten Träger-, Hilfs- und Zusatzstoffen sowie in Kombination mit Nahrungsergänzungsstoffen. Die Komponenten können in räumlich getrennten Zubereitungen verabreicht werden. Es ist darüber hinaus auch möglich, diese in einer Formulierung anzuwenden, mit der entsprechend fallbezogenen Konzentrationen der Einzelkomponenten zur systemischen Anwendung, wie z.B. oral, bukkal, pulmonal, nasal, transdermal, intravenös, subcutan, intracutan, intramuskulär, rektal, vaginal und intrathekal, zusammen mit an sich bekannten Träger-, Hilfs- und Zusatzstoffen sowie in Kombination mit Nahrungsergänzungsstoffen verabreicht werden.

Besonders geeignet sind die zwei oder mehr der oben im Detail genannten Komponenten enthaltenden pharmazeutischen Zubereitungen gemäß der Erfindung im Rahmen der Prophylaxe und Behandlung degenerativer Erkrankungen des ZNS. Dabei kann die Behandlung gleichzeitig, in getrennten Formulierungen oder auch zeitlich abgestuft erfolgen. Mit Vorteil finden die pharmazeutischen Zubereitungen gemäß der Erfindung Verwendung bei der Prophylaxe und Therapie folgender Erkrankungen: ischämischer und hämorrhagischer Schlaganfall, Amyotrophe Lateralsklerose, Alzheimer'sche Erkrankung, Parkinson'sche Erkrankung, Hunntington'sche Erkrankung, Multiple Sklerose, Neurodegeneration im Alter, Demenz, Schädel-Hirntrauma und Autosomal Dominanter Neurohypophysealer Diabetes Insipidus (ADNDI). In weiteren Ausführungsformen findet die erfindungsgemäße pharmazeutische Zubereitung auch Verwendung bei der Prävention und Therapie von zerebraler Ischämie infolge von kardialen und kardiovaskulären Insulten.

Die erfindungsgemäßen pharmazeutischen Zubereitungen mit einer Kombination von zwei oder mehr Substanzen aus der Gruppe α-Liponsäure, Ambroxol und Inhibitor des Angiotensin-Converting-Enzyms und ihre Verwendung im Rahmen der Prophylaxe und Therapie degenerativer Erkrankungen des ZNS wird anhand der Beispiele und Abbildungen näher beschrieben. Die Angabe der Beispiele erfolgt nur zu Zwecken der Veranschaulichung bevorzugter Ausführungsformen der Erfindung und dient nicht der Beschränkung der Erfindung.

### Beispiel 1:

In Beispiel 1 ist der neuartige und überraschende Befund dargestellt, wonach neuronale Zellen unter physiologischen Bedingungen über deutlich weniger freie Thiolgruppen verfügen als nichtneuronale Zellen. Die Markierung freier Thiolgruppen erfolgte mit dem Fluoreszenzfarbstoff 5-(und 6-)-(((4-Chlormethyl-) benzoyl-) amino-) tetramethylrhodamine (CMTMR). Die reaktive Chlormethylgruppe von CMTMR reagiert dabei zelltypunabhängig mit freien Thiolgruppen von Proteinen, wodurch diese nach spezifischer Anregung im UV-Bereich sichtbar gemacht werden.

Figur 1 zeigt ein Übersichtsbild an organotypischen hippokampalen Gewebeschnitten. Überraschenderweise zeigen gerade die ausgesprochen vulnerablen Neuronen der Hippokampusformation (Pyramidenzellen) einen sehr geringen und damit kaum detektierbaren Gehalt an freien Thiolgruppen.

Diese Spezifität tritt ganz deutlich in den Figuren 1b und 2b zutage. Hier sind die Ergebnisse einer Doppelmarkierung dargestellt. Neben einem spezifischen Marker für freie Thiolgruppen (CMTMR) wurde NeuroTrace (NT) als neuronaler Marker eingesetzt. Figur 2a zeigt dabei die Fluoreszenz von CMTMR (rot) und NT (grün). Beide Fluroreszenzsignale sind zellulär nicht kolokalisiert, was belegt, daß NT und CMTMR weitgehend in unterschiedlichen Zelltypen, nämlich in neuronalen bzw. nicht-neuronalen Zellen lokalisiert sind. Die Figuren 1b und 2b zeigen die Überlagerung beider Kanäle.

### Beispiel 2

### Einfluß der Substanzkombination auf das Überleben neuronaler Zellen nach neurodegenerativen Ereignissen

Zur Untersuchung der neuroprotektiven Wirkung der thiolaktiven Substanzen wurde das in unserem Labor standardisierte Verfahren der transienten Ischämie an organotypischen hippokampalen Gewebeschnitten verwendet [14-16]. Dabei handelt es sich um dreidimensionale kultivierte Explantate mit einem hohen Vorhersagewert für nachfolgende klinische Untersuchungen. Bei diesen Gewebekulturen, die sowohl neuronale als auch nicht-neuronale Zellen enthalten, sind hochkomplexe interzelluläre Verbindungen weitgehend erhalten. Diese sind für die Funktionalität des gesamten ZNS von außerordentlicher Bedeutung. Die zerebralen Gewebekulturen wurden einem degenerativen Ereignis ausgesetzt, welches analog zu klinischen Situationen zum Untergang neuronaler Zellen führt.

Die Gewebeschnitte (375 µm) wurden anschließend aus dem Hippokampus von Ratten (P7) präpariert. Die Kultivierung dieser Schnittkulturen fand auf Membraneinsätzen der Porengröße 0,02 µm statt. Nach 10 bis 12 Tagen Kultivierung (37°C im Inkubator; 3,3 % CO₂; 98 % relative Luftfeuchte) waren Restrukturierungsprozesse innerhalb der Schnittkulturen abgeschlossen und an den Gewebeschnitten wurde durch einen transienten Sauerstoff/Glukoseentzug klinisch relevantes neurodegeneratives Ereignis simuliert. Die Substanzwirkung wurde gemessen an der Veränderung des Standardschadens (Vehikelapplikation).

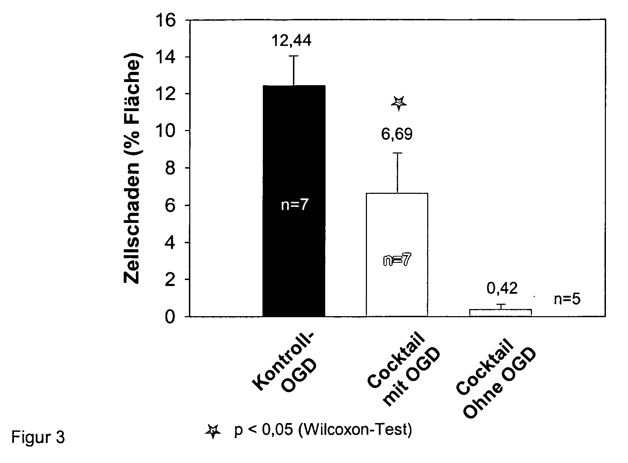

In Figur 3 ist die Wirkung der Kombination von α-Liponsäure (10 µg/ml), Ambroxol (10 µM) und Enalapril (20 µg/ml) (im folgenden als "Cocktail" bezeichnet) auf das Überleben von neuronalen Zellen nach OGD dargestellt. Quantifiziert wurde die neuronale Schädigung nach einer Standard-OGD mit und ohne Cocktail-Applikation sowie die Schädigung durch Cocktail *per se* (eventuelle Substanztoxizität). Tabelle 1 zeigt zusammengefaßt die statistischen Parameter der entsprechenden Stichproben. Der Schaden wird ausgedrückt in % geschädigte Fläche. Dazu wurde für jeden Gewebeschnitt die gesamte Fläche des Schnittes ins Verhältnis zur geschädigten Fläche gesetzt.

**Tabelle 1**

| **Spezifität** | **Anzahl** | **Mittelwert** | **Median** | **SEM** |
|---|---|---|---|---|
| Kontroll-OGD | 7 | 12,45 | 12,8 | 1,59 |
| | | | | |
| Kontroll-OGD + Cocktail | 7 | 6,69 | 7,73 | 2,1 |
| | | | | |
| Cocktail ohne OGD | 5 | 0,42 | 0,08 | 0,23 |

Überraschenderweise konnte durch die Applikation der erfindungsgemäßen und in den Patentansprüchen beanspruchten beanspruchten Kombination aus den oben näher spezifizierten Komponenten eine ausgeprägte und signifikante Verminderung des neuronalen Schadens erzielt werden. Beziffert wird dieser neuroprotektive Effekt auf ca. 40 bis 45 %. Gleichzeitig belegt dieses Experiment, daß die getestete Kombination ohne degeneratives Ereignis zu keiner Schädigung von neuronalen Zellen führt. Dies stellt eine elementare Voraussetzung für die klinische Anwendung zur Therapie bzw. Prävention von neurodegenerativen Erkrankungen dar.

### Beispiel 3

### Vergleich der Wirkung der kombinierten Anwendung der erfindungsgemäß beanspruchten Kombination mit der Wirkung der Einzelkomponenten

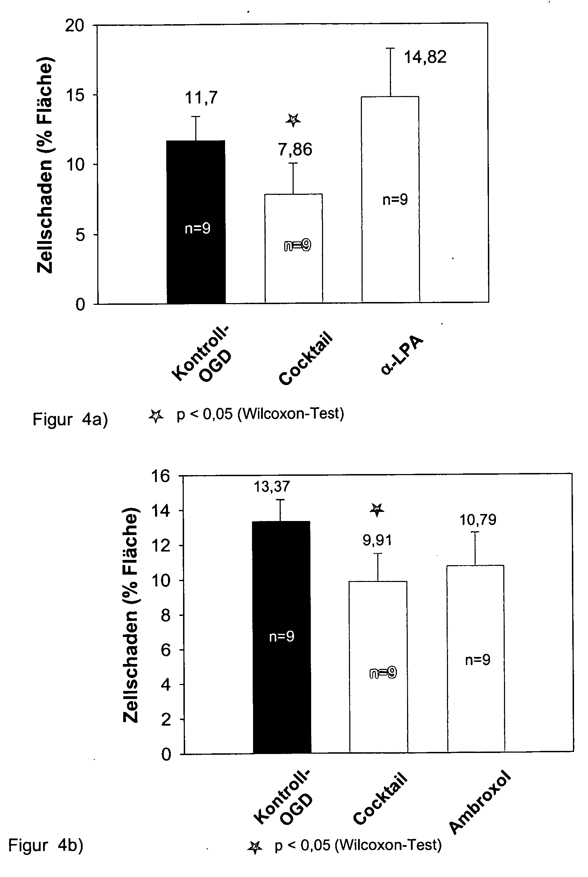

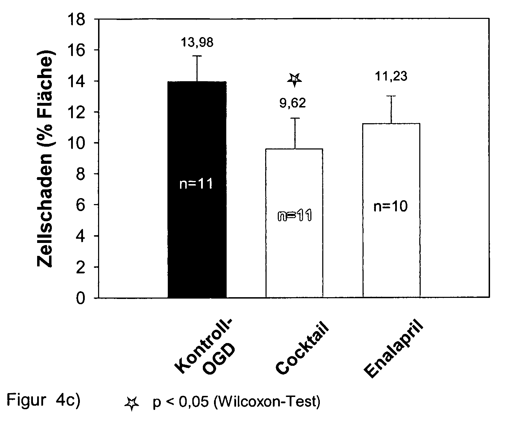

In diesen Versuchen wurde nachgewiesen, daß die Einzelsubstanz-Applikation zu keinem signifikanten neuroprotektiven Effekt führt. Den Versuchen liegt das unter Beispiel 2 beschriebene Protokoll zugrunde. Bei den in den Figuren 4a bis c dargestellten Versuchen wurde der protektive Effekt des Cocktails mit dem Standardschaden nach OGD und dem Effekt der entsprechenden Einzelsubstanz verglichen. Aus allen Experimenten geht deutlich hervor, daß keine der drei getesteten Substanzen allein den Schaden nach Neurodegeneration mindert, wohingegen der Cocktail in der Lage ist, signifikanten Schutz zu vermitteln. Tabelle 2a bis c zeigt die relevanten statistischen Parameter.

**Tabelle 2a)**

| **Spezifität** | **Anzahl** | **Mittelwert** | **Median** | **SEM** |
|---|---|---|---|---|
| Kontroll-OGD | 9 | 11,70 | 10,8 | 1,71 |
| | | | | |
| Kontroll-OGD + Cocktail | 9 | 7,86 | 5,53 | 2,18 |
| | | | | |
| Kontroll-OGD + α-Liponsäure | 9 | 14,82 | 13,54 | 3,40 |

**Tabelle 2b)**

| **Spezifität** | **Anzahl** | **Mittelwert** | **Median** | **SEM** |
|---|---|---|---|---|
| Kontroll-OGD | 9 | 13,37 | 11,99 | 1,22 |
| | | | | |
| Kontroll-OGD + Cocktail | 9 | 9,91 | 8,65 | 1,57 |
| | | | | |
| Kontroll-OGD + Ambroxol | 9 | 10,79 | 8,32 | 1,88 |

**Tabelle 2c)**

| **Spezifität** | **Anzahl** | **Mittelwert** | **Median** | **SEM** |
|---|---|---|---|---|
| Kontroll-OGD | 11 | 13,98 | 12,27 | 1,63 |
| | | | | |
| Kontroll-OGD + Cocktail | 11 | 9,62 | 7,57 | 1,95 |
| | | | | |
| Kontroll-OGD + Enalapril | 10 | 11,23 | 8,70 | 1,76 |

Im Gegensatz zum Cocktail (Zubereitung enthaltend α-Liponsäure, Ambroxol und ACE-Inhibitor) waren weder α-Liponsäure noch Ambroxol noch der ACE-Inhibitor bei Einzelapplikation in der Lage, den neuronalen Schaden nach transienter OGD signifikant zu erniedrigen.

### Literaturverweise:

1. White, B.C., et al., *Fluorescent histochemical localization of lipid peroxidation during brain reperfusion following cardiac arrest*. Acta Neuropathol (Berl), 1993. **86**(1): p. 1-9.
2. Munch, G., et al., *Advanced glycation end products in neurodegeneration: more than early markers of oxidative stress?* Ann Neurol, 1998. **44**(3 Suppl 1): p. S85-8.
3. Koppal, T., et al., *Peroxynitrite-induced alterations in synaptosomal membrane proteins: insight into oxidative stress in Alzheimer's disease.* J Neurochem, 1999. **72**(1): p. 310-7.
4. Wang, X., F. Manganaro, and H.M. Schipper, *A cellular stress model for the sequestration of redoxactive glial iron in the aging and degenerating nervous system.* J Neurochem, 1995. **64**(4): p. 1868-77.
5. Liu, R.M., *Down-regulation of gammaglutamylcysteine synthetase regulatory subunit gene expression in rat brain tissue during aging.* J Neurosci Res, 2002. **68**(3): p. 344-51.
6. Iciek, M., M. Polak, and L. Wlodek, *Effect of thiol drugs on the oxidative hemolysis in human erythrocytes.* Acta Pol Pharm, 2000. **57**(6): p. 449-54.
7. Kenchappa, R.S., et al., *Thioltransferase (glutaredoxin) mediates recovery of motor neurons from excitotoxic mitochondrial injury.* J Neurosci, 2002. **22**(19): p. 8402-10.
8. Trotti, D., N.C. Danbolt, and A. Volterra, *Glutamate transporters are oxidant-vulnerable: a molecular link between oxidative and excitotoxic neurodegeneration?* Trends Pharmacol Sci, 1998. **19**(8): p. 328-34.
9. Packer, L., E.H. Witt, and H.J. Tritschler, *alpha-Lipoic acid as a biological antioxidant.* Free Radic Biol Med, 1995. **19**(2): p. 227-50.
10. Gillissen, A. and D. Nowak, *Characterization of N-acetylcysteine and ambroxol in anti-oxidant therapy*. Respir Med, 1998. **92**(4): p. 609-23.
11. Teramoto, S., et al., *Effects of ambroxol on spontaneous or stimulated generation of reactive oxygen species by bronchoalveolar lavage cells harvested from patients with or without chronic obstructive pulmonary diseases*. Pharmacology, 1999. **59**(3): p. 135-41.
12. Gibbs, B.F., et al., *Ambroxol inhibits the release of histamine, leukotrienes and cytokines from human leukocytes and mast cells.* Inflamm Res, 1999. **48**(2): p. 86-93.
13. Jablonka, S., et al., *The influence of Ambroxol on peroxidative processes in lung and plasma in dogs after pulmonectomy.* Arch Vet Pol, 1992. **32**(1-2): p. 57-66.
14. Stoppini, L., P.A. Buchs, and D. Muller, *A simple method for organotypic cultures of nervous tissue.*
   J Neurosci Methods, 1991. **37**(2): p. 173-82.
15. Breder, J., et al., *Organotypic hippocampal slice cultures as an in vitro model for the investigation of neuroprotective drugs against ischemic damage.* Schriften des Forschungszentrums Jülich, 1999. **3**.
16. Striggow, F., et al., *The protease thrombin is an endogenous mediator of hippocampal neuroprotection against ischemia at low concentrations but causes degeneration at high concentrations.* Proc Natl Acad Sci U S A, 2000. **97**(5): p. 2264-9.

## Patentansprüche

1. Pharmazeutische Zubereitungen, umfassend zwei oder mehr Substanzen aus der Gruppe α-Liponsäure und deren Salze und Isomere, Ambroxol und dessen Salze und Prodrugs und Inhibitoren des Angiotensin-Converting-Enzyms (ACE), gegebenenfalls zusammen mit üblichen pharmazeutisch annehmbaren Träger-, Zusatz- und Hilfsstoffen.

2. Pharmazeutische Zubereitungen nach Anspruch 1, umfassend eine Kombination aus α-Liponsäure oder deren Salzen oder Isomeren und Ambroxol oder dessen Salzen oder dessen Prodrugs, oder umfassend eine Kombination aus α-Liponsäure oder deren Salzen oder Isomeren und mindestens einem Inhibitor des Angiotensin-Converting-Enzyms, oder umfassend eine Kombination aus Ambroxol oder dessen Salzen oder dessen Prodrugs und mindestens einem Inhibitor des Angiotensin-Converting-Enzyms, oder umfassend eine Kombination aus α-Liponsäure oder deren Salzen oder Isomeren und Ambroxol oder dessen Salzen oder dessen Prodrugs und mindestens einem Inhibitor des Angiotensin-Converting-Enzyms.

3. Pharmazeutische Zubereitungen nach einem der Ansprüche 1 bis 2, umfassend zusätzlich einen oder mehrere bekannte Träger-, Hilfs- und/oder Zusatzstoffe.

4. Pharmazeutische Zubereitungen nach einem der Ansprüche 1 bis 3, umfassend α-Liponsäure oder deren Salze oder Isomere in Mengen im Bereich von 30 bis 1.200 mg/d, bevorzugt im Bereich von 200 bis 600 mg/d, und/oder Ambroxol oder dessen Salze oder dessen Prodrugs in Mengen im Bereich von 7,5 bis 90 mg/d, bevorzugt im Bereich von 60 bis 75 mg/d, und/oder mindestens einen Inhibitor des Angiotensin-Converting-Enzyms in Mengen im Bereich von 1 bis 50 mg/d, bevorzugt im Bereich von 5 bis 20 mg/d.

5. Pharmazeutische Zubereitungen nach einem der Ansprüche 1 bis 4 zur oralen, bukkalen, pulmonalen, nasalen, transdermalen, intravenösen, subcutanen, intracutanen, intramuskulären, rektalen, vaginalen und intrathekalen Verabreichung, bevorzugt in Form von Tabletten, Pulvern, Granulaten, Kapseln, Lösungen, Emulsionen, Suspensionen, Aerosolen, transdermalen Applikationssystemen, Zäpfchen und Darreichungsformen mit retardierter Abgabe einzelner oder aller Wirkstoffe.

6. Verwendung der pharmazeutischen Zubereitungen nach einem oder mehreren der Ansprüche 1 bis 5 zur Herstellung eines Arzneimittels zur Prävention und Therapie von neurodegenerativen Erkrankungen.

7. Verwendung nach Anspruch 6 zur Herstellung eines Arzneimittels zur Prävention und Therapie der Erkrankungen ischämischer und hämorrhagischer Schlaganfall, Amyotrophe Lateralsklerose, Alzheimer'sche Erkrankung, Parkinson'sche Erkrankung, Hunntington'sche Erkrankung, Multiple Sklerose, Neurodegeneration im Alter, Demenz, Schädel-Hirntrauma und Autosomal Dominanter Neurohypophysealer Diabetes Insipidus.

8. Verwendung nach Anspruch 6 zur Prävention und Therapie von zerebraler Ischämie infolge von kardialen und kardiovaskulären Insulten.
